# EUROPÄISCHE PATENTSCHRIFT

(11) **EP 0 534 097 B1**
(45) Veröffentlichungstag und Bekanntmachung des Hinweises auf die Patenterteilung: **19.03.1997**
(21) Anmeldenummer: 92113288.2
(22) Anmeldetag: 04.08.1992
(51) Int. Cl.: A61K 31/70

(54) **Verwendung von NADH oder NADPH gegen Krebs oder AIDS**
Use of NADH or NADPH against cancer or AIDS
Utilisation de NADH ou NADPH contre le cancer ou le SIDA

(30) Priorität: 26.08.1991 DE 4128625
(43) Veröffentlichungstag der Anmeldung: 31.03.1993
(73) Patentinhaber: Birkmayer, Jörg, Univ.-Prof. DDr., A-1090 Wien (AT)
(72) Erfinder: Birkmayer, Jörg, Univ.-Prof. DDr., A-1090 Wien (AT)
(74) Vertreter: von Hellfeld, Axel, Dr. Dipl.-Phys.

(56) Entgegenhaltungen:
- EP-A- 0 135 955
- EP-A- 0 331 620

## Beschreibung

Die Erfindung betrifft die neuartige Verwendung von Nikotinamid-adenin-dinukleotid (NADH), Nikotinamid-adenin-dinukleotidphosphat (NADPH) oder eines physiologisch verträglichen Salzes derselben.

Die Erscheinungsformen von Krebserkrankungen sind vielfältig und hinsichtlich ihrer Genese in vielen Fällen zumindest noch nicht vollständig aufgeklärt, wobei neben genetischer Disposition je nach Art des Tumors auch bestimmte Risikofaktoren eine Rolle zu spielen scheinen. Die Therapie von Krebserkrankungen beruht im wesentlichen auf den drei Säulen: Operation, Bestrahlung und Chemotherapie, die oft in Kombination zur Anwendung kommen und (ebenfalls je nach Tumortyp) unterschiedliche Erfolgsraten aufweisen. In jedem Fall stellen alle diese Therapieformen einen massiven Eingriff mit allen damit zusammenhängenden Risiken und Nebenwirkungen dar.

Das erstmals 1981 als eigenständiges Krankheitsbild beschriebene Immundefektsyndrom AIDS (acquired immune deficieny syndrome) ist charakterisiert durch erst im fortgeschrittenen Krankheitsstadium auftretende persistierende oder rezidivierende Erkrankungen, die auf Defekte im zellulären Immunsystem hinweisen und durch bestimmte Retroviren hervorgerufen werden. Dabei treten bei dieser Erkrankung in der Spätphase fast immer auch maligne Karzinome oder Sarkome auf, wie bspw. das Kaposi-Sarkom.

Eine spezifische Therapie von Aids ist zur Zeit noch nicht bekannt. Die Behandlung beschränkt sich noch darauf, die lebensbedrohlichen Infektionen und Neoplasmen soweit wie möglich zu bekämpfen. Zum Einsatz kommen verschiedene antivirale Substanzen, wie bspw. AZT (Azidodeoxythymidin) oder Stimulatoren des Immunsystems, wie bspw. Interferone oder Interleukine.

Es besteht daher ein großes Bedürfnis nach einem Medikament, mit dessen Hilfe bösartige Tumore bei Mensch und Tier und klinisch-manifeste AIDS-Erkrankungen kontrolliert, gehemmt und unterdrückt werden können. Es ist daher Aufgabe der Erfindung, ein derartiges Therapeutikum zur Verfügung zu stellen.

Die Erfindung betrifft die Verwendung von Nikotinamid-Adenin-Dinukleotid (NADH), Nikotinamid-Adenin-Dinukleotidphosphat (NADPH) oder eines physiologisch verträglichen Salzes derselben zur Herstellung eines Arzneimittels zur Behandlung von Krebs und zur Behandlung von bösartigen Tumoren bei AIDS-Erkrankungen. Vorzugsweise werden die Einzeldosen von NADH oder NADPH bzw. eines physiologisch verträglichen Salzes derselben 1 mg bis 50 mg betragen. Noch bevorzugter kommen Einzeldosen von 5 mg bis 12,5 mg zum Einsatz.

Der Einsatz dieser körpereigenen Substanzen führt zu überraschenden therapeutischen Erfolgen, ohne daß Nebenwirkungen zu erkennen oder zu erwarten sind. Es ist zwar bekannt, daß NADH erfolgreich bei der Behandlung des Parkinson-Syndroms (EP-A-0 331 620) eingesetzt werden kann. Weiterhin ist aus der EP-A-0 135 955 bekannt, daß man spontane bösartige Tumore durch Verabreichung von NADH zusammen mit Pyridoxal-5-phosphat behandeln kann. Die völlig überraschende Erkenntnis, daß die Wirkstoffe NADH bzw. NADPH in den oben angegebenen Dosierungen auch äußerst erfolgreich bei der Behandlung von bösartigen Tumoren und AIDS eingesetzt werden können, hat sich aus den im folgenden detailliert beschriebenen klinischen Befunden ergeben. Dabei konnte bewiesen werden, daß NADH und NADPH bzw. physiologisch verträgliche Salze derselben die Metastasenbildung hemmen bzw. Remissionen herbeiführen und daß sich unter diesen Substanzen die zellulären Immunreaktionen verbessern und damit zu einer Rückbildung der klinischen AIDS-Symptome führen.

### FALLBESCHREIBUNGEN

- Fall 1:: Weibliche Patientin, 63 Jahre, August 1989 Operation wegen eines duktalen invasiven Mammakarzinoms. 1 Jahr später Nachweis von multiplen Leber- und Knochenmetastasen. 4 Therapiezyklen, nach dem CMF-Schema, weitere Zunahme der Leber- und Knochenmetastasen. Schmerzzustände nur mit stärksten Analgetika einzudämmen. Seit Januar 1991 NADH, zunächst dreimal wöchentlich 12,5 mg, intravenös, nach 4-wöchiger parenteraler Therapie Umstellung auf NADH oral 5 mg jeden zweiten Tag. April 1991 röntgenologischer Nachweis der Metastasenrückbildung, einige Herde stark verkleinert, einige komplett verschwunden. Fortsetzung der oralen NADH-Therapie, Kontrolle Juni 1991, Computertomogrammbefund zeigt eine weitere deutliche Rückbildung der Lebermetastasen, Knochenmetastasen fast nicht mehr nachweisbar. Patientin ist schmerzfrei und benötigt keinerlei Analgetika mehr.
- Fall 2:: Männlicher Patient, 59 Jahre, vor drei Jahren Colonkarzinom, 1990 sonographischer und radiologischer Nachweis von multiplen Lebermetastasen von Kirsch- bis Pflaumengröße. 2 Chemotherapiezyklen, Myleran bzw. Endoxan Zeigen kein Ansprechen, Leberherde nehmen an Größe zu. Dezember 1990 Beginn der Therapie mit NADH, zunächst 12,5 mg intravenös dreimal wöchentlich, nach vierwöchiger Therapieumstellung auf NADH oral 5 mg jeden zweiten Tag. März 1991 sonographischer Nachweis der Verkleinerung der Leberherde. Juni 1991 Kontrolle durch Computertomographie und Sonographie zeigt ein fast völliges Verschwinden der Absiedlungen in der Leber. Patient fühlt sich subjektiv ausgezeichnet.
- Fall 3:: Weibliche Patientin, 52 Jahre, vor drei Jahren Quadrantenektomie wegen invasiven szirrhösem Mammakarzinom, Januar 1990 Nachweis von Wirbelsäulenmetastasen, April 1990 Auffinden von Lebermetastasen durch Ultraschall. Therapie mit Novaldex zeigt bei den Metastasen keine Rückbildungstendenz. Ein Therapiezyklus nach dem CMF-Schema spricht ebenfalls nicht an. November 1990 Anwendung von NADH intravenös 12,5 mg jeden zweiten Tag. Nach 4 Wochen Umstellung auf NADH oral 5 mg jeden zweiten Tag. Zwei Monate nach Beginn der NADH-Therapie deutliche Rückbildung der Lebermetastasen sowie Verschwinden bzw. Verkleinerung der Wirbelsäulenmetastasen. Kontrolle Juni 1991 Knochenabsiedelungen vollkommen rückgebildet, Lebermetastasen stark verkleinert bzw. Herde nicht mehr erkennbar.
- Fall 4:: Männlicher Patient, 66 Jahre, Februar 1990 Diagnose eines kleinzelligen Bronchialkarzinoms, multiple Herde in beiden Lungenlappen, zytostatische Therapie mit Methotrexat und Endozan führt zu keiner Remission. Oktober 1990 Anwendung von NADPH parenteral (10 mg intravenös) jeden zweiten Tag. Röntgenologische Kontrolle im Januar 1991 zeigt Rückgang der neoplastischen Herde in Zahl und Größe, Fortsetzung der NADPH-Therapie 10 mg i.v. jeden zweiten Tag, Kontrolle im Mai 1991 durch Computertomogramm bestätigt eine weitere Verkleinerung und Verringerung der Tumorherde in beiden Lungenlappen.
- Fall 5:: Männlicher Patient, 42 Jahre, HIV positiv, Western Plot positiv, seit November 1989 klinisch manifestes AIDS mit Entwicklung eines Kaposisarkoms. Therapie mit AZT: Stabilisierung des Sarkomwachstums für 3 Monate, dann trotz fortgesetzter AZT-Therapie Auftreten von neuen zusätzlichen Sarkomherden, die operativ entfernt werden mußten. 6 Wochen später neuerliches Auftreten von Tumorgewebe im Operationsbereich, aber auch an alternierenden Stellen. Fortsetzung der AZT-Therapie. Seit Dezember 1990 Anwendung von NADH, 1. Phase 12,5 mg intravenös jeden zweiten Tag, nach 4 Wochen Umstellung auf NADH oral 5 mg täglich. Nach acht Wochen deutlicher Rückgang der neugebildeten Geschwülste, Kontrolle nach 4 Monaten zeigt keinerlei Neubildung im Behandlungsintervall, fast völlige Rückbildung der Sarkomresiduen.
- Fall 6:: Männlicher Patient, 28 Jahre, seit Ende 1989 klinische Symptome von AIDS, rezidivierende Infekte, Pneumonien, Herpes- und Pilzinfektionen. HIV positiv, Western Plot positiv, seit Mitte 1990 unter AZT-Therapie, keine wesentliche Besserung des Zustandes während sechsmonatiger Anwendung. Seit Februar 1991 Therapie mit NADPH 10 mg intravenös jeden zweiten Tag, Sistieren der Infekte, derzeit keine Pneumonien und Pilzinfektionen, Laboruntersuchungen zeigen Abnahme der T-Supressor-Zellen, Zunahme des Verhältnis von T4 und T8 Zellen, zellulärer Immunstatus deutlich besser. HIV und Western Plot nach wie vor positiv.

Die in der vorstehenden Beschreibung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verwendung von Nikotinamid-Adenin-Dinukleotid (NADH), Nikotinamid-Adenin-Dinukleotidphosphat (NADPH) oder eines physiologisch verträglichen Salzes derselben zur Herstellung eines Arzneimittels zur Behandlung von Krebs und zur Behandlung von bösartigen Tumoren bei AIDS-Erkrankungen.

2. Verwendung nach Anspruch 1, wobei die Einzeldosen von 1 mg bis 50 mg betragen.

3. Verwendung nach Anspruch 2, wobei die Einzeldosen 5 mg bis 12,5 mg betragen.

## Claims

1. Use of nicotinamide-adenine dinucleotide (NADH), nicotinamide-adenine dinucleotide phosphate (NADPH) or a physiologically compatible salt thereof for the preparation of a medicament for the treatment of cancer and for the treatment of malignant tumours with AIDS.

2. Use according to claim 1 wherein the single doses are 1 mg to 50 mg.

3. Use according to claim 2 wherein the single doses are 5 mg to 12,5 mg.

## Revendications

1. Utilisation de nicotinamide-adénine-dinucléotide (NADH), de nicotinamide-adénine-dinucléotide-phosphate (NADPH) ou d'un sel physiologiquement toléré de ces derniers pour la préparation d'un médicament destiné au traitement du cancer et au traitement de tumeurs malignes dans les aspects cliniques du SIDA.

2. Utilisation selon la revendication 1, les doses uniques étant de 1 mg à 50 mg.

3. Utilisation selon la revendication 2, les doses uniques étant de 5 mg à 12,5 mg.
